# EUROPEAN PATENT SPECIFICATION

(11) **EP 1 361 901 B1**
(45) Date of publication and mention of the grant of the patent: **24.01.2007**
(21) Application number: 02711008.9
(22) Date of filing: 04.02.2002
(51) Int. Cl.: A61L 9/01, A61L 9/14

(54) **MACROCYCLIC MUSKS FOR SMOKE MALODOUR PREVENTION AND COUNTERACTION**
MAKROZYKLISCHE MOSCHUSMISCHUNGEN ZUR VERHINDERUNG ODER VERRINGERUNG VON RAUCHGERÜCHEN
MUSCS MACROCYCLIQUES DESTINES A PREVENIR OU A DIMINUER LES MAUVAISES ODEURS DES FUMEES

(30) Priority: 20.02.2001 GB 0104094
(43) Date of publication of application: 19.11.2003
(73) Proprietor: Quest International Services B.V., 1411 GP Naarden (NL)
(72) Inventor: DUPREY, Roger John Henry, Canterbury, Kent, CT4 7BS (GB); MICHAEL, Loren, Hall, Freehold, NJ 07728 (US); PAYNE, Ian Michael, Willesborough, Ashford, Kent TN24 0RY (GB); PERRING, Keith Douglas, Ashford, Kent TN24 8HS (GB); POPE, Alexander Spencer, Rochester, Kent, ME1 1SQ (GB); ELLWOOD, Simon, Brabourne Lees, Ashford, Kent TN25 6QE (GB); RICHARDSON, Anne, Canterbury, Kent CT4 7TA (GB)
(74) Representative: Matthews, Heather Clare
(86) International application number: PCT/GB2002/000501
(87) International publication number: WO 2002/066083

(56) References cited:
- US-A- 6 077 318
- US-A- 6 103 679
- PATENT ABSTRACTS OF JAPAN vol. 018, no. 068 (C-1161), 4 February 1994 (1994-02-04) & JP 05 279690 A (KANEBO LTD), 26 October 1993 (1993-10-26)
- PATENT ABSTRACTS OF JAPAN vol. 2000, no. 09, 13 October 2000 (2000-10-13) & JP 2000 154394 A (LION CORP), 6 June 2000 (2000-06-06)

## Description

### Field of the Invention

The present invention relates to the use of certain perfume ingredients to prevent and/or counteract smoke malodours, particularly tobacco smoke malodour. The invention also relates to perfumes and products which incorporate such perfume ingredients.

### Background to the Invention

The problem of preventing and/or counteracting malodours has been recognised for many years, and numerous methods and products have been developed to address specific malodours where these occur. A recent addition to the array of methods and products available, has been the development and mass marketing of several consumer products designed for general use in household and industrial malodour eradication.

Fragrance materials, typically as part of a perfume (also known as a fragrance), are probably the most commonly used malodour counteractants either alone or in combination with other materials such as antibacterial agents or absorbents.

Perfumes are known which are effective against a range of malodours such as body odour for example, but perfumes which demonstrate to good effect, an activity against smoke malodour are rare.

WO 95/15186 describes the use of certain perfumery aldehydes, or mixtures thereof, and perfumes containing them, for counteracting smoke malodours in enclosed air spaces or on substrates on which smoke malodour has deposited. The perfumery aldehydes are described as effectively counteracting tobacco odour.

FR 2666510 discloses deodorising compositions designed to counteract malodours from many disparate sources, e.g. animal and vegetable sources, industrial processes, drains and tobacco combustion. A number of perfumery aldehydes, alcohols and esters together with recommended dosages are detailed, among which citral and phenylacetaldehyde are key components. Further components, such as those described as a 'chemical molecule' (as distinct presumably from similar materials used in perfumery) are required to be present, preferably in at least 5 % by weight of the composition; the sole example of this class is the straight chain aldehyde heptanal. Additionally, the compositions comprise from 0.5 to 10% of an agent which equalises olfactive perception e.g. xylene musks or tetralin. Several air freshening product formulations are disclosed as embodiments of the invention.

WO 98/32820 concerns perfumes, and products for treating skin, hair and textile fibres, which comprise a mixture of at least two of the macrocyclic musks; hexadecanolide, cyclopentadecanone and pentadecanolide. The musk mixtures of WO 98/32820 are described as possessing good substantivity to cellulosic and proteinaceous substrates as well as being easily biodegradable. The mixtures are thus stated to be useful replacements for polycyclic musks such as Tonalid^{™} which may have good substantivity, but poor biodegradability. Perfumes may comprise from 0.01%, preferably at least 0.1% and more preferably at least 0.5% by weight or more of the described musk mixtures.

JP 05-279690 discloses a perfume composition containing one or more of ylang-ylang, lavender and lavandine that masks cigarette odour. Reference is made to other perfume materials, including cyclopentadecanolide, but not in relation to masking of cigarette odour.

### Summary of the Invention

According to one aspect of the present invention, there is provided use of one or more macrocyclic musks selected from cyclohexadecanolide, cyclopentadecanone or cyclopentadecanolide in a composition for preventing and/or counteracting smoke malodours on a surface.

For the purposes of the present invention, the expression "preventing smoke malodours on a surface" means that the application to a smoke free surface of one or more musks according to the invention, typically reduces or suppresses the olfactory detection of the evolution of smoke malodour from the surface when it comes into contact with smoke.

For the purposes of the present invention, the expression "counteracting smoke malodours on a surface" means that the application to a surface exhibiting smoke malodour of one or more musks in accordance with the invention, typically reduces the perceived intensity of smoke malodour evolved from the surface.

The present invention is based on the appreciation that one or more biodegradable macrocyclic musks selected from cyclohexadecanolide, cyclopentadecanone or cyclopentadecanolide may be used to conveniently provide a cost effective and surprisingly efficacious means for preventing and/or counteracting smoke malodours, particularly tobacco malodour, on a surface. Advantageously, the musks are particularly effective in preventing and/or counteracting smoke malodours on a fabric surface, such as cloth. Furthermore, compositions comprising comparatively high levels of a musk, or mixtures thereof, may demonstrate at least a three fold reduction in the perceived smoke malodour intensity on a surface compared with a control. It has also been found that, generally, owing to the substantive properties attributed to one or more of the above defined macrocyclic musks, the smoke malodour counteractancy and/or preventative effect observed from a particular musk or musk mixture, is advantageously not merely ephemeral but may persist for an extended period of time.

The composition may be optionally admixed with further excipients. Preferably, the composition is in the form of a perfume. For the purposes of the present invention, "a perfume" is defined as containing one or more further perfume ingredients, if desired mixed with a suitable solvent, in addition to the one or more macrocyclic musks selected from cyclohexadecanolide, cyclopentadecanone or cyclopentadecanolide.

There are a number of possible mechanisms by which the one or more macrocyclic musks as defined herein may prevent and/or counteract smoke malodour. For example, the one or more macrocyclic musks may partially neutralise the olfactory perception of smoke malodour, this effect arising in part from the phenomena of spatial and/or temporal filtering. In spatial filtering, it is possible that the one or more macrocyclic musks may compete with the molecules responsible for smoke malodour for odour receptor sites or cells at the olfactory epithelium, or they may evoke intracellular effects which may inhibit transduction processes that occur during olfactory stimulation. Alternatively, through a process of temporal filtering, the magnitude and content of the neural message produced within the nose may be reduced through inhibition at the olfactory bulb and other central structures by the presence of the one or more macrocyclic musks.

### Macrocyclic Musks

As noted above, the macrocyclic musks that have been found to be efficacious in a composition for preventing and/or counteracting smoke malodour, particularly tobacco odour, are one or more macrocyclic musks selected from cyclohexadecanolide, cyclopentadecanone or cyclopentadecanolide.

Preferred for use herein is a composition comprising a mixture of at least two of cyclohexadecanolide, cyclopentadecanone and cyclopentadecanolide. More preferred for use herein is a mixture comprising cyclohexadecanolide and cyclopentadecanone.

Typically, the weight ratio of cyclohexadecanolide to cyclopentadecanone in such a mixture is from about 1:3 to about 10:1, and preferably about 1:1.

Preferably, compositions useful herein additionally comprise one or more further perfume ingredients.

In a preferred embodiment of the present invention, when a composition comprising a mixture of at least two of cyclohexadecanolide, cyclopentadecanone and cyclopentadecanolide is applied to a surface exhibiting smoke malodour, it has been found that such mixtures possess substantivity to cellulosic and proteinaceous surfaces which is at least as good as, but in most cases significantly better, than the substantivity of the separate components cyclohexadecanolide, cyclopentadecanone and cyclopentadecanolide to such surfaces. This potentially enhanced substantivity of such mixtures thus constitutes an additional benefit. Furthermore, compositions comprising a mixture of at least two of the aforementioned macrocyclic musks have been found to be particularly efficacious in preventing and/or counteracting smoke malodour and without being limited by any particular theory, it is thought that the efficacy of a particular mixture may result from a synergistic effect occurring between the macrocyclic musks.

The compositions useful herein can be a perfume as defined hereinabove, or a product which comprises, for example, a perfume which is additionally admixed with other excipients such as, for example, suitably compatible solvents, cleaning agents or humectants etc.

The one or more macrocyclic musks, for example, may be part of a complete perfume which apart from the musk odour imparts many other desirable olfactory properties to the perfume.

Malodour counteracting perfumes useful in the invention generally comprise at least 3%, preferably at least 10%, more preferably at least 15%, and even more preferably at least 20% by weight of the perfume of the one or more macrocyclic musks.

In perfumes, one or more macrocyclic musks may be blended with many other perfume ingredients known in the art to obtain a harmonious total odour of which the musk odour is one aspect. Therefore, a perfume is typically a mixture of volatile materials exhibiting a variety of odour characters, which in combination, are pleasing to the human nose.

Thus the invention provides in a further aspect, a perfume comprising at least 10%, preferably at least 15% and most preferably at least 20% by weight of the perfume of two or more macrocyclic musks selected from cyclohexadecanolide, cyclopentadecanone or cyclopentadecanolide and one or more further perfume ingredients.

Other perfume ingredients that may be advantageously combined with a macrocyclic musk or mixtures thereof according to the invention in a perfume are, for example, certain natural extracts, essential oils, absolutes, resinoids, resins, concretes etc., but also synthetic materials such as hydrocarbons, alcohols, aldehydes, ketones, ethers, acids, esters, acetals, ketals, nitriles, etc., including saturated and unsaturated compounds, aliphatic, carbocyclic and heterocyclic compounds.

Such perfume ingredients are mentioned, for example, in S. Arctander, Perfume and Flavor Chemicals (Montclair, N.J., 1969), in S. Arctander, Perfume and Flavor Materials of Natural Origin (Elizabeth, N.J., 1960) and in "Flavor and Fragrance Materials - 1997", Allured Publishing Co. Wheaton, Ill. USA, or earlier versions of this yearly publication.

Examples of perfume ingredients which can be used in combination with the one or more macrocyclic musks in accordance with the invention are: geraniol, geranyl acetate, linalol, linalyl acetate, tetrahydrolinalol, citronellol, citronellyl acetate, geranyl nitrile available under the trade mark "CITRALVA", dihydromyrcenol, dihydromyrcenyl acetate, tetrahydromyrcenol, terpineol, terpinyl acetate, nopol, nopyl acetate, 2-phenylethanol, 2-phenylethyl acetate, benzyl alcohol, benzyl acetate, benzyl salicylate, styrallyl acetate, benzyl benzoate, amyl salicylate, dimethylbenzyl-carbinol, trichloromethylphenylcarbinyl acetate, p-tert-butylcyclohexyl acetate, isononyl acetate, vetiveryl acetate, vetiverol, α-hexylcinnamaldehyde, 2-methyl-3-(p-tert-butylphenyl)propanal, 2-methyl-3-(p-isopropylphenyl)propanal, 3-(p-tert-butylphenyl)-propanal, 2,4-dimethylcyclohex-3-enyl-carboxaldehyde, tricyclodecenyl acetate, tricyclodecenyl propionate, 4-(4-hydroxy-4-methylpentyl)-3-cyclohexenecarboxaldehyde, 4-(4-methyl-3-pentenyl)-3-cyclohexenecarboxaldehyde, 4-acetoxy-3-pentyl-tetrahydropyran, 3-carboxymethyl-2-pentylcyclopentane, 2-n-heptylcyclopentanone, 3-methyl-2-pentyl-2-cyclopentenone, n-decanal, n-dodecanal, 9-decen-1-ol, phenoxyethyl isobutyrate, phenylacetaldehyde dimethyl acetal, phenylacetaldehyde diethyl acetal, citronellyl nitrile, cedryl acetate, 3-isocamphylcyclohexanol, cedryl methyl ether, aurantion, 2-ethyl-4(2', 2', 3'-trimethylcyclopent-3'-enyl)but-2-enol available under the trade mark "BANGALOL", isolongifolanone, aubepine nitrile, aubepine, heliotropin, coumarin, eugenol, ethyl vanillin, vanillin, diphenyl oxide, hydroxy-citronellal, ionones, methylionones, isomethylionones, irones, 3a,4,5,6,7,7a-hexahydro-4,7-methano-1(3)H-inden-6-ylacetate available under the trade mark "JASMACYCLENE", lavandin grosso, lily aldehyde, lixetone, methyl naphthyl ketone, moss oak moss, orange oil, patchouli acid, petitgrain oil, rose oxide, 2,4-dimethyl-4-phenyltetrahydrofuran available under the trade mark "RHUBAFURAN", dicyclopentenyl pivalate available under the trade mark "PIVACYCLENE", cis-3-hexenol and esters thereof, indan musks, tetralin musks, isochroman musks.

The compositions or perfumes in accordance with the present invention typically comprise a solvent, in which the one or more macrocyclic musks and/or further perfume ingredients are diluted. Suitable solvents for use herein are compatible with the one or more macrocyclic musks, as well as with the surface to which the one or more macrocyclic musks are applied. A suitable example of a solvent useful herein includes isopropyl myristate.

When performing the process of the invention, the composition is either, applied to a surface exhibiting smoke malodour to counteract the smoke malodour, or the composition is applied to a surface free of smoke malodour to typically prevent the surface exhibiting smoke malodour, including surfaces such as skin, hair, a hard surface, fabric, floor covering, or wall paper. The composition may be applied by spraying, for instance, as an aerosol or by a pump action, by wiping, for instance from an impregnated cloth, pouring etc.

Perfumes according to the invention, can be utilised in products suitable for application to a surface which has been in contact with smoke and which thus exhibits smoke malodour, or to a surface which may come into contact with smoke malodour thereby typically preventing the surface exhibiting smoke malodour. The invention in an even further aspect therefore provides a product comprising a perfume in accordance with the invention.

Suitable products include for example, hard surface cleaners, laundry detergents: including liquid, granular and tablet forms, fabric softeners, rinse conditioners, fabric treatment products including fabric refresher products, e.g. sprays, starch sprays, ironing sprays and stain remover sprays, shampoos, conditioners, mousse, hair sprays, hair refresher sprays and lotions and the like, creams, lotions, face and body spritzers, gels, bath and shower products, face washes, solutions and the like. A preferred product is a fabric refresher product.

In a further aspect the invention provides a process for preventing and/or counteracting smoke malodours on a surface, comprising applying to the surface a composition comprising two or more macrocyclic musks selected from cyclohexadecanolide, cyclopentadecanone or cycopentadecanolide.

The invention is illustrated by way of the following non-limiting examples.

All percentages are by weight unless otherwise indicated.

For brevity and simplicity in the Examples, the macrocyclic musks, cyclohexadecanolide and cyclopentadecanolide, will be referred to by their commonly used abbreviated terminology in the fragrance industry of "hexadecanolide" and "pentadecanolide" respectively.

### Example 1

The reduction in the perceived smoke malodour intensity from a surface exhibiting smoke malodour following the application of a composition comprising one or more macrocyclic musks in accordance with the invention to the surface, was demonstrated as described below using standard sensory protocols.

Perfumes (A-F), each comprising a core fragrance base to which was added various macrocyclic and polycyclic musks, were prepared in the proportions indicated in the tables below.

**Core fragrance base**

| **Ingredient** | **% w/w** |
|---|---|
| Aurantion (Q) | 0.4 |
| Bangalol (Q) | 2 |
| Benzyl Acetate Extra (Q) | 0.6 |
| Benzyl Salicylate (Q) | 16 |
| Citralva (IFF) | 0.2 |
| Citronellol Pure | 4 |
| Diphenyl Oxide | 0.6 |
| Dipropylene Glycol | 2.3 |
| Ethyl Vanillin | 0.4 |
| Eugenol Rectified | 0.6 |
| Geranyl Acetate | 0.6 |
| Hexyl Cinnamic Aldehyde | 10 |
| Jasmacyclene (Q) | 2 |
| Lavandin Grosso | 5 |
| Lily Aldehyde | 12 |
| Linalol | 1.4 |
| Lixetone Bulked (Q) | 16 |
| Methyl Naphthyl Ketone | 1.2 |
| Moss Oakmoss Synthetic | 0.4 |
| Orange Oil | 0.8 |
| Patchouli Acid Washed (Q) | 5.6 |
| Petitgrain Paraguay | 1.2 |
| Pivacyclene (Q) | 0.2 |
| Rhubafuran (Q) 10% In DPG | 0.4 |
| Rose Oxide Racemic | 0.1 |

- Perfume ingredients identified by (Q) are commercially available from Quest International, Ashford, Kent, UK.
- Perfume ingredients identified by (IFF) are commercially available from International Fragrances and Flavourings, New York City, USA.

| **Perfume** | **A** | **B** | **C** | **D** | **E** | **F** |
|---|---|---|---|---|---|---|
| Core fragrance base | 84 | 84 | 84 | 84 | 84 | 84 |
| MIX-S¹ | | | | | 16 | |
| Tonalid ™ ² | 16 | | | | | |
| Pentadecanolide | | 16 | | | | |
| Hexadecanolide | | | 16 | | | |
| Cyclopentadecanone | | | | 16 | | |
| Habanolide ³ | | | | | | 16 |
| Total | 100 | 100 | 100 | 100 | 100 | 100 |

1. MIX - S¹ is a mixture consisting of 35% hexadecanolide, 35% cyclopentadecanone and 30% isopropyl myristate.
2. Tonalid is the Trade Mark for a polycyclic musk commercially available from PFW Aoma Chemicals B.V., The Netherlands.
3. Habanolide is the Trade Mark for a macrolide musk commercially available from Firmenich S.A., Geneva, Switzerland.

Six fabric refresher sprays including one of perfumes A, B, C, D, E or F above were prepared according to the formulation indicated below:

**Fabric Refresher Spray**

| **Ingredient** | **% w/w** |
|---|---|
| Perfume (A, B, C, D, E or F) | 0.05 |
| Solubiliser ⁴ | 0.15 |
| Preservative as required | |
| Purified water | up to 100 |

### 4. Cremaphor RH40, commercially available from BASF AG.

Seven terry towelling squares (10cm x 10cm) were labelled and placed into a smoking cabinet of internal dimensions 37 x 48 x 59 cm containing two Marlboro cigarettes for 20 minutes, after which time, the cloths were removed from the cabinet. Six of the cloths were each sprayed with 1.5g of one of the six fabric refresher sprays and left to dry for 2 hours. A control cloth sample was also included in the test which was sprayed with a non-perfumed fabric refresher spray. The individual cloths were then bagged in odour free plastic bags and labelled with random three figure numbers ready for assessment.

### Odour Assessment

### Sensory Panel

A trained sensory panel of assessors selected on the basis of their sensory acuity were used to assess the odour intensity of the cloths. The panellists were trained for a period of 6 months prior to the tests being carried out to develop their ability to accurately and reproducibly score their perceived intensity of odours. Once training was successfully completed, they were regularly employed on a part time basis to carry out sensory assessments.

The cloth samples were presented to the panellists in a randomised order. Each assessor individually scored each of the cloths for perceived intensity of smoke malodour using a magnitude estimation scaling technique as described in "Sensory Evaluation Techniques", Meilgaard, Civille and Carr; CRC Press (1991); pg 55 and references cited therein. The individual panellist scores were normalised and analysed using standard statistical Analysis of Variance and Multiple Comparison tests, to produce an average malodour intensity score.

The results are shown in the table below:

| **Perfume** | **Malodour Intensity Score** |
|---|---|
| B | 2 |
| E | 3 |
| C | 5 |
| D | 11 |
| F | 13 |
| A | 32 |
| Control (perfume free) | 88 |

It can be seen from the results indicated in the table above, that whilst all of the tested perfumes effect a reduction in the perceived intensity of smoke malodour compared with a control, fabric refresher sprays which include a perfume comprising one or more biodegradable macrocyclic musks demonstrate to varying degrees improved smoke malodour counteractancy properties compared with fabric refresher sprays comprising perfumes containing either one of the fragrance industry's high tonnage non-biodegradable musks, Tonalid^{™} (perfume A), or the macrolide musk Habanolide ^{™} (perfume F).

### Example 2

The smoke malodour counteractancy properties (as measured by perceived smoke malodour intensity) of a macrocyclic musk mixture comprising hexadecanolide and cyclopentadecanone varies with the concentration of the musk mixture in a perfume as detailed below.

Perfumes (G-I) containing a 70% solution of equal amounts of hexadecanolide and cyclopentadecanone in isopropyl myristate (ie. 35% hexadecanolide: 35% cyclopentadecanone: 30% isopropyl myristate) additionally admixed with various proportions of a core fragrance base (of the same composition as indicated in Example 1) were prepared as indicated in the table below:

| **Perfume** | **G** | **H** | **I** |
|---|---|---|---|
| Core fragrance base | 70% | 84% | 95% |
| Musk mixture | 30% | 16% | 5% |
| of which | | | |
| Hexadecanolide/cyclopentadecanone | 21% | 11.2% | 3.5% |
| Isopropyl myristate | 9% | 4.8% | 1.5% |

Three fabric refresher sprays including one of perfumes G, H or I were prepared according to the formulation indicated below:

**Fabric Refresher Spray**

| **Ingredient** | **% w/w** |
|---|---|
| Perfume (G, H or I) | 0.03 |
| Solubiliser (as in Example 1 above) | 0.15 |
| Preservative as required | |
| Purified Water | up to 100 |

Four terry towelling squares (10cm x 10cm) were labelled and placed into a smoking cabinet of internal dimensions 37 x 48 x 59 cm containing two Marlboro cigarettes for 20 minutes, after which time, the cloths were removed from the cabinet. Each of three cloths was sprayed with 1.5g of one of the three fabric refresher sprays and left to dry for 2 hours. The remaining cloth sample was used as a control and was left untreated. The individual cloths were then bagged in odour free plastic bags and labelled for assessment.

The cloth samples were assessed for their perceived intensity of smoke malodour using the procedure described in Example 1.

The results are shown in the table below:

| **Perfume** | **% Musk mixture in perfume** | **Malodour Intensity Score** |
|---|---|---|
| G | 30% | 13 |
| H | 16% | 16 |
| I | 5% | 28 |
| Control (untreated) | - | 95 |

The results indicate that perfumes comprising comparatively high levels of a mixture of the macrocyclic musks, hexadecanolide and cyclopentadecanone, demonstrate good smoke malodour counteractancy properties.

### Example 3

The following example demonstrates the observed variation in smoke malodour counteractancy properties from fabric refresher sprays comprising perfumes with differing ratios of a macrocyclic musk mixture containing hexadecanolide, cyclopentadecanone and isopropyl myristate.

Perfumes J, H and K containing 84% core fragrance base (as described in Example 1) and 16% of a 70% solution of varying amounts of hexadecanolide and cyclopentadecanone in 30% isopropyl myristate were prepared as indicated below:

| **Perfume** | **Hexadecanolide:** | **Cyclopentadecanone:** | **Isopropyl myristate** |
|---|---|---|---|
| J | 63 | 7 | 30 |
| H | 35 | 35 | 30 |
| K | 21.5 | 48.5 | 30 |

Three fabric refresher sprays including one of perfumes J, H or K were prepared according to the base formulation indicated in Example 2. Following which, each of the fabric refresher sprays were sprayed onto cloth squares exhibiting smoke malodour. As in Example 2, an untreated cloth square exhibiting smoke malodour was also included for assessment. The cloth samples were then assessed for their perceived intensity of smoke malodour using the procedures described in Example 2.

The results are indicated in the table below:

| **Perfume** | **Ratio Hexadecanolide:Cyclopentadecanone** | **Malodour Intensity Score** |
|---|---|---|
| J | 9:1 | 9 |
| H | 1:1 | 12 |
| K | 4:9 | 24 |
| Control (untreated) | - | 95 |

The results indicate that smoke malodour counteractancy benefits are delivered by perfumes comprising musk mixtures of differing ratios.

## Claims

1. Use of one or more macrocyclic musks selected from cyclohexadecanolide, cyclopentadecanone or cyclopentadecanolide in a composition for preventing and/or counteracting smoke malodours on a surface.

2. A process for preventing and/or counteracting smoke malodours on a surface comprising applying to the surface a composition comprising two or more macrocyclic musks selected from cyclohexadecanolide, cyclopentadecanone or cyclopentadecanolide.

3. A process according to claim 2, wherein the mixture comprises cyclohexadecanolide and cyclopentadecanone.

4. A process according to claim 3, wherein the weight ratio of cyclohexadecanolide to cyclopentadecanone is from 1:3 to 10:1.

5. A process according to claim 4, wherein the weight ratio of cyclohexadecanolide to cyclopentadecanone is about 1:1.

6. A process according to any one of claims 2 to 5, wherein the composition is a perfume.

7. A process according to claim 6, wherein the perfume comprises at least 3 % by weight of the perfume of the one or more macrocyclic musks.

8. A process according to claim 7, wherein the perfume comprises at least 10% by weight of the perfume of the one or more macrocyclic musks.

9. A process according to any one of claims 2 to 8, wherein the surface is skin, hair, a hard surface, fabric, floor covering or wall paper.

10. A perfume comprising at least 10% by weight of the perfume of two or more macrocyclic musks selected from cyclohexadecanolide, cyclopentadecanone or cyclopentadecanolide, and one or more further perfume ingredients.

11. A perfume according to claim 10, wherein the mixture comprises cyclohexadecanolide and cyclopentadecanone.

12. A product comprising a perfume according to claim 10 or claim 11.

## Patentansprüche

1. Verwendung einer makrocyclischen Moschusverbindung oder mehrerer makrocyclischer Moschusverbindungen, ausgewählt aus Cyclohexadecanolid, Cyclopentadecanon und Cyclopentadecanolid, in einer Zusammensetzung zur Verhinderung und/oder Bekämpfung von Rauchgerüchen an einer Oberfläche.

2. Verfahren zur Verhinderung und/oder Bekämpfung von Rauchgerüchen auf einer Oberfläche, umfassend das Auftragen einer Zusammensetzung, die zwei oder mehr makrocyclische Moschusverbindungen, ausgewählt aus Cyclo-hexadecanolid, Cyclopentadecanon und Cyclopentadecanolid, umfasst, auf die Oberfläche.

3. Verfahren nach Anspruch 2, wobei das Gemisch Cyclohexadecanolid und Cyclopentadecanon umfasst.

4. Verfahren nach Anspruch 3, wobei das Gewichtsverhältnis von Cyclohexadecanolid zu Cyclopentadecanon 1:3 bis 10:1 ist.

5. Verfahren nach Anspruch 4, wobei das Gewichtsverhältnis von Cyclohexadecanolid zu Cyclopentadecanon etwa 1:1 ist.

6. Verfahren nach einem der Ansprüche 2 bis 5, wobei die Zusammensetzung ein Parfüm ist.

7. Verfahren nach Anspruch 6, wobei das Parfüm mindestens 3 Gew.-% des Parfüms aus der einen oder den mehreren makrocyclischen Moschusverbindung(en) umfasst.

8. Verfahren nach Anspruch 7, wobei das Parfüm wenigstens 10 Gew.-% des Parfüms aus einen oder den mehreren makrocyclischen Moschusverbindung(en) umfasst.

9. Verfahren nach einem der Ansprüche 2 bis 8, wobei die Oberfläche Haut, Haar, eine harte Oberfläche, Stoff, Bodenbelag oder Tapete ist.

10. Parfüm, umfassend wenigstens 10 Gew.-% des Parfüms aus zwei oder mehr makrocyclischen Moschusverbindungen, ausgewählt aus Cyclohexadecanolid, Cyclopentadecanon und Cyclopentadecanolid, und ein oder mehrere weitere Parfüm-Ingredienzien.

11. Parfüm nach Anspruch 10, wobei das Gemisch Cyclohexadecanolid und Cyclopentadecanon umfasst.

12. Produkt, das ein Parfüm nach Anspruch 10 oder 11 umfasst.

## Revendications

1. Utilisation d'un ou plusieurs muscs macrocycliques choisis parmi le cyclohexadécanolide, la cyclopentadécanone ou le cyclopentadécanolide dans une composition pour prévenir et/ou neutraliser les mauvaises odeurs de fumée sur une surface.

2. Procédé pour prévenir et/ou neutraliser les mauvaises odeurs de fumée sur une surface comprenant l'application sur la surface d'une composition comprenant deux muscs macrocycliques ou plus choisis parmi le cyclohexadécanolide, la cyclopentadécanone ou le cyclopentadécanolide.

3. Procédé selon la revendication 2, dans lequel le mélange comprend le cyclohexadécanolide et la cyclopentadécanone.

4. Procédé selon la revendication 3, dans lequel le rapport en poids du cyclohexadécanolide sur la cyclopentadécanone est de 1/3 à 10/1.

5. Procédé selon la revendication 4, dans lequel le rapport en poids du cyclohexadécanolide sur la cyclopentadécanone est d'environ 1/1.

6. Procédé selon l'une quelconque des revendications 2 à 5, dans lequel la composition est un parfum.

7. Procédé selon la revendication 6, dans lequel le parfum comprend au moins 3 % en poids du parfum des un ou plusieurs muscs macrocycliques.

8. Procédé selon la revendication 7, dans lequel le parfum comprend au moins 10 % en poids du parfum des un ou plusieurs muscs macrocycliques.

9. Procédé selon l'une quelconque des revendications 2 à 8, dans lequel la surface est une peau, des cheveux, une surface dure, un tissu, un revêtement de sol ou un papier mural.

10. Parfum comprenant au moins 10 % en poids du parfum de deux muscs macrocycliques ou plus choisis parmi le cyclohexadécanolide, la cyclopentadécanone ou le cyclopentadécanolide et un ou plusieurs autres ingrédients de parfum.

11. Parfum selon la revendication 10, dans lequel le mélange comprend le cyclohexadécanolide et la cyclopentadécanone.

12. Produit comprenant un parfum selon la revendication 10 ou la revendication 11.
